Europäisches Patentamt

European Patent Office

Office européen des brevets

(11) Veröffentlichungsnummer: **0 407 920 A1**

## EUROPÄISCHE PATENTANMELDUNG

(21) Anmeldenummer: **90112995.7**

(22) Anmeldetag: **07.07.90**

(51) Int. Cl.5: **A61M 5/168**, G01F 25/00

(30) Priorität: **12.07.89 DE 3922952**

(43) Veröffentlichungstag der Anmeldung:
**16.01.91 Patentblatt 91/03**

(84) Benannte Vertragsstaaten:
**DE ES FR GB IT**

(71) Anmelder: **Fresenius AG**
**Gluckensteinweg 5**
**D-6380 Bad Homburg v.d.H.(DE)**

(72) Erfinder: **Polaschegg, Hans-Dietrich, Dr.**
**Grünweisenweg 9**
**D-6370 Oberursel 4(DE)**

(74) Vertreter: **Dr. Fuchs, Dr. Luderschmidt**
**Dipl.-Phys. Seids, Dr. Mehler Patentanwälte**
**Abraham-Lincoln-Strasse 7, Postfach 4660**
**D-6200 Wiesbaden(DE)**

(54) **Verfahren und Anordnung zum Kalibrieren des Tropfvolumens tropfengeregelter Druckinfusionsapparate.**

(57) Zum Kalibrieren des Tropfenvolumens tropfengeregelter Druckinfusionsapparate, die in Kombination mit volumetrisch arbeitenden Druckinfusionsapparaten oder Durchflußmessern betrieben werden, wird die den tropfengeregelten Druckinfusionsapparat durchlaufende Flüssigkeit dem volumetrisch arbeitenden Druckinfusionsapparat oder Durchflußmesser zugeführt und die während des Betriebs dieser Einrichtungen ermittelte Tropfenzahl pro Zeiteinheit mit dem Volumen pro Zeiteinheit verglichen. Aus dem Vergleich der beiden Werte wird auf das Tropfenvolumen geschlossen. Eine Anordnung zur Durchführung des Verfahrens weist einen tropfengeregelten Druckinfusionsapparat (2) und einen volumetrisch arbeitenden Druckinfusionaapparat (1) oder einen Durchflußmesser (18) auf, die ausgangsseitig in einer gemeinsamen Flüssigkeitsleitung (8) münden, in der eine Absperreinrichtung (4) angeordnet ist. Die beiden Apparate (1) und (2) sind an eine Auswerte-Einheit (3) angeschlossen. Zur Kalibrierung des tropfengeregelten Druckinfusionsapparates (1) wird die gemeinsame Flüssigkeitsleitung (8) unterbrochen und die volumetrisch arbeitende Pumpe (1) in umgekehrter Richtung betrieben, so daß aus dem tropfengeregelten Zweig (9) die Flüssigkeit in den Zweig (10) gesaugt wird.

FIG.1

## VERFAHREN UND ANORDNUNG ZUM KALIBRIEREN DES TROPFVOLUMENS TROPFENGEREGELTER DRUCKINFUSIONSAPPARATE

Die Erfindung betrifft ein Verfahren und eine Anordnung zum Kalibrieren des Tropfenvolumens tropfengeregelter Druckinfusionsapparate, die in Kombination mit volumetrischen Druckinfusionsapparaten oder Durchflußmessern betrieben werden.

Bei der Infusionstherapie besteht naturgemäß das Bestreben, ein Arzneimittel präzise zu dosieren. Der Wirkstoff ist dabei in der Regel in vorgegebener Menge in einem vorgegebenen Volumen enthalten. Um den Wirkstoff in der vorgegebenen Menge zu verabreichen, muß ein definiertes Volumen infundiert werden.

Zu diesem Zweck werden volumetrisch arbeitende Pumpen, in der Regel peristaltisch arbeitende Schlauchpumpen eingesetzt. Um die erforderliche Präzision zu erreichen, muß allerdings der Schlauchinnendurchmesser eng toleriert sein ( + - 2-5%). Diese hohen Anforderungen an die Schläuche machen diese vergleichsweise aufwendig und teuer.

Pumpen oder Regler, die mit Hilfe eines Tropfendetektors gesteuert werden, das heißt bei denen die Anzahl der Tropfen pro Zeiteinheit vorgegeben wird, erlauben dagegen die Verwendung einfacher, preisgünstiger Schläuche, wie sie ansonsten für die apparatefreie Schwerkraftinfusion eingesetzt werden.

Das Volumen eines Tropfens unterliegt Schwankungen von bis zu 400%, die von den Toleranzen des tropfenbildenden Elementes, der Viskosität und der Oberflächenspannung der Flüssigkeit abhängen. Die genaue Applikation beliebiger Arzneimittel oder Arzneimittelgemische ist mit einem tropfengeregelten Druckinfusionsapparat nicht möglich. Lediglich eine einmal eingestellte Tropfenrate kann stabil gehalten werden.

Aus der DE-PS 36 37 771 ist eine Infusionseinrichtung bekannt, die über einen Füllstandssensor zur Kalibrierung einer Infusionspumpe verfügt. Das Tropfenvolumen eines tropfengeregelten Druckinfusionsapparates wird nicht kalibriert.

In dem G 87 13 337.7 wird ein Druckinfusionsapparat beschrieben, bei dem aus einem Infusionsbehälter die Flüssigkeit über einen Tropfenzähler einer Pumpe zugeführt wird. In der Flüssigkeitsleitung zwischen Tropfenzähler und Pumpe sind zwei Sensoren angeordnet, mit denen die Förderrate der Pumpe bestimmt wird. Anhand des Resultats wird dann die Pumpe gesteuert.

Aus der DE-OS 24 19 520 ist eine Infusionseinrichtung bekannt, bei der die Tropfenzahl in einem Tropfglied erfaßt und einer Steuereinrichtung zugeführt wird, die dann einen Quetschstempel ansteuert. Eine Kalibrierung des Tropfensteuers wird nicht vorgenommen.

Aus der DE-PS 37 02 609 ist ein Druckinfusionsapparat bekannt, bei dem die zu infundierende Flüssigkeit aus einem an einer Waage hängenden Behälter über ein Tropfsystem einer Pumpe zugeführt wird. Einerseits wird das Ausgangssignal der Waage in ein Flüssigkeitsvolumen umgerechnet und mit einem Sollwert verglichen, woraus ein Signal für die Korrektur der Pumpendrehzahl gebildet wird. Andererseits wird die Tropfenzahl auf optischem Wege im Tropfensystem ermittelt und unter Berücksichtigung der Dichte in ein Volumen umgerechnet, das ebenfalls mit dem Sollwert verglichen wird. Bei deutlichen Abweichungen wird die Pumpe stillgesetzt und ein Alarm ausgelöst. Da das Tropfenvolumen jedoch nicht kalibriert ist, ist auch das Ergebnis dieses Überwachungskanals mit einem Fehler behaftet.

Aufgabe der Erfindung ist ein Verfahren und eine Anordnung, mit der die Genauigkeit der Infusionsmenge bei tropfengeregelten Druckinfusionsapparaten verbessert werden kann.

Diese Aufgabe wird mit einem Verfahren gemäß den kennzeichnenden Merkmalen von Anspruch 1 und einer Anordnung gemäß den kennzeichnenden Merkmalen von Anspruch 6 gelöst.

Es hat sich überraschend herausgestellt, daß eine größere Genauigkeit tropfengeregelter Druckinfusionsapparate dadurch erreicht werden kann, daß das Tropfenvolumen tropfengeregelter Druckinfusionsapparate kalibriert wird, was besonders einfach dann durchgeführt werden kann, wenn die in den Infusionsapparaten vorhandenen volumetrischen Druckinfusionsapparate oder Durchflußmesser zur Bestimmung des Tropfenvolumens eingesetzt werden.

Zur Kalibrierung wird die den tropfengeregelten Druckinfusionsapparat durchlaufende Flüssigkeit dem volumetrischen Druckinfusionsapparat oder Durchflußmesser zugeführt. Beide Vorrichtungen werden in Betrieb genommen und die an dem tropfengeregelten Druckinfusionsapparat ermittelte oder eingestellte Tropfenzahl pro Zeiteinheit mit dem an dem volumetrischen Druckinfusionsapparat oder Durchflußmesser ermittelten oder eingestellten Volumen pro Zeiteinheit verglichen. Aus dem Verhältnis von Volumen pro Zeiteinheit zu Tropfenzahl pro Zeiteinheit wird anschließend das Tropfenvolumen erhalten. Dies ist ein besonders einfaches und wirtschaftliches Verfahren, das es ermöglicht, auch tropfengeregelte Druckinfusionsapparate dort einzusetzen, wo es auf präzise Dosierung ankommt.

Wenn der tropfengeregelte Druckinfusionsapparat und der volumetrische Druckinfusionsapparat ausgangsseitig an eine gemeinsame Flüssigkeitsleitung angeschlossen sind, wird zur Kalibrierung des Tropfenvolumens der volumetrische Druckinfusionsapparat in umgekehrter Richtung betrieben. Derartige Apparatekombinationen finden sich bei Mehrfachinfusionseinrichtungen, die Spritzenpumpen, Infusionspumpen wie z.B. peristaltische Pumpen oder Infusionsregler aufweisen. Die beiden von den Einrichtungen abgehenden Schläuche werden mit Hilfe eines Abzweigstückes (T-Stück, Y-Stück) miteinander verbunden. Von dieser Verbindung geht ein drittes, zum Patienten führendes Schlauchstück ab.

Die gemeinsame Flüssigkeitsleitung kann entweder durch eine Klemme oder durch ein Ventil geschlossen werden.

Zur Kalibrierung des Tropfenvolumens des tropfengeregelten Druckinfusionsapparates wird zunächst das gesamte System luftfrei gefüllt. Anschließend wird die gemeinsame Flüssigkeitsleitung unterbrochen, was durch das Ventil oder die Klemme bewerkstelligt wird. Der volumetrische Druckinfusionsapparat wird sodann in umgekehrter Förderrichtung in Betrieb genommen, wobei die Förderrate vorgegeben ist und somit bekannt ist. Handelt es sich bei dem tropfengeregelten Druckinfusionsapparat um einen Infusionsregler, so wird durch die Wirkung der Pumpe Flüssigkeit aus dem tropfengeregelten Zweig abgesaugt. Dadurch fallen Tropfen im tropfengeregelten Zweig, deren Zahl pro Zeiteinheit erfaßt wird. Da das Volumen pro Zeiteinheit des volumetrischen Druckinfusionsapparates bekannt ist, kann durch Division das Verhältnis Tropfenzahl zu Volumen und das Volumen pro Tropfen bestimmt werden.

In der Folge kann diese für eine Infusionsbehandlung konstante Größe zur Umrechnung einer vorgegebenen volumetrischen Infusionsrate in Tropfen pro Zeiteinheit verwendet werden.

Das Verfahren läßt sich entsprechend auch dann anwenden, wenn eine Kombination aus einem volumetrischen Infusionsregler und einer tropfengeregelten Pumpe vorliegt. In diesem Fall wird die tropfengeregelte Pumpe zunächst mit einer vorgegebenen Tropfenrate betrieben und danach die Förderrichtung umgekehrt. Dabei wird das Volumen pro Zeiteinheit in dem in normaler Förderrichtung betriebenen Regler gemessen.

Das Verfahren ist auch anwendbar, wenn es sich um zwei Pumpen handelt. Hierbei muß sichergestellt werden, daß die Pumprate der tropfengeregelten Pumpe auf die Pumprate der volumetrisch arbeitenden Pumpe eingestellt ist. Der Nachweis kann mit einem in der gemeinsamen Flüssigkeitsleitung liegenden Flußsensor oder durch einen im Bereich zwischen den beiden Pumpen und dem Ventil liegenden Drucksensor erfolgen.

Im ersten Fall wird die tropfengeregelte Pumpe mit vorgegebener Rate betrieben und die volumetrische Pumpe so geregelt, daß bei umgekehrter Förderrichtung dieser Pumpe der Nettofluß in der gemeinsamen Flüssigkeitsleitung Null wird. Die Förderraten beider Pumpen sind dann gleich. Auf die gleiche Weise kann auch die Rate der volumetrischen Pumpe vorgegeben und die Rate der tropfengeregelten Pumpe nachgeregelt werden.

Im zweiten Fall werden die Pumpraten so aufeinander abgestimmt, daß bei geschlossenem Ventil in der gemeinsamen Flüssigkeitsleitung der Druck im System konstant bleibt.

Das erfindungsgemäße Verfahren ist dann auch einsetzbar, wenn ein volumetrischer Druckinfusionsapparat einem tropfengeregelten Druckinfusionsapparat nachgeschaltet ist. Insbesondere bei Mehrfachinfusionseinrichtungen können mehrere tropfengeregelte Druckinfusionsapparate an eine gemeinsame Patientenleitung angeschlossen sein, in der sich ein volumetrischer Druckinfusionsapparat oder ein Durchflußmesser befindet.

Handelt es sich dabei um einen kalibrierten Druckinfusionsapparat, so kann bei dem Mehrfachinfusionssystem jeder tropfengeregelte Druckinfusionsapparat dadurch kalibriert werden, daß er initial einzeln betrieben und seine Tropfenrate mit dem gemessenen Fluß (Volumen/Zeiteinheit) verglichen und somit kalibriert wird.

Ist der Durchflußmesser nicht kalibriert, so kann er mit Hilfe einem weiteren volumetrischen Druckinfusionsapparates initial kalibriert werden, und dann dazu verwendet werden, die übrigen angeschlossenen Einrichtungen zu kalibrieren.

Das erfindungsgemäße Verfahren kann auch auf die Kombination mehrerer tropfengeregelter Druckinfusionsapparate mit einem einzigen volumetrischen Druckinfusionsapparat erweitert werden. Auf diese Weise können auch Multi-Infusionsapparate kalibriert werden, deren einzelne Kanäle tropfengeregelt sind, wobei lediglich eine einzige volumetrische Pumpe zur Förderung und damit zur Kalibrierung herangezogen wird.

Die erfindungsgemäße Anordnung zur Durchführung des Verfahrens ist dadurch gekennzeichnet, daß der Ausgang des tropfengeregelten Druckinfusionsapparates an den Ausgang oder an den Eingang des volumetrischen Druckinfusionsapparates oder Durchflußmessers ohne Zwischenschaltung weiterer Meßgeräte angeschlossen ist und daß beide Apparate elektrisch an eine Auswerte-Einheit angeschlossen sind, die zur Ermittlung des Tropfenvolumens aus der Tropfenzahl pro Zeiteinheit und des Volumens pro Zeiteinheit ausgebildet ist.

Der tropfengeregelte Druckinfusionsapparat kann ein Infusionsregler oder eine Pumpe sein. Der volumetrische Druckinfusionsapparat kann ebenfalls eine Pumpe oder ein Infusionsregeler sein.

Vorzugsweise können auch mindestens zwei tropfengeregelte Druckinfusionsapparate ausgangsseitig an eine gemeinsame Flüssigkeitsleitung angeschlossen sein, wobei der volumetrische Druckinfusionsapparat oder Durchflußmesser in der gemeinsamen Flüssigkeitsleitung angeordnet ist.

Gemäß einer weiteren Ausführungsform sind der tropfengeregelte Druckinfusionsapparat und der volumetrische Druckinfusionsapparat ausgangsseitig an die gemeinsame Flüssigkeitsleitung angeschlossen, in der eine Absperreinrichtung vorgesehen ist. Anstelle der Absperreinrichtung kann auch ein Flußsensor oder ein Drucksensor angeordnet sein.

Der Vorteil des Verfahrens bzw. der Anordnung besteht darin, daß auf einfache Weise der meist bereits in Infusionsapparaten vorhandene volumetrische Druckinfusionsapparat oder Durchflußmesser zur Kalibrierung des Tropfenvolumens des tropfengeregelten Druckinfusionsapparates eingesetzt werden kann. Zusätzlich in der Flüssigkeitsleitung eingesetzte Meßgeräte sind somit nicht erforderlich.

Beispielhafte Ausführungsformen der Erfindung werden nachfolgend anhand der Zeichnung näher erläutert.

Es zeigen

Figur 1 eine schematische Darstellung der Anordnung

Figur 2 eine schematische Darstellung einer anderen Ausführungsform

Figur 3 die schematische Darstellung einer noch anderen Ausführungsform

Figur 4 die schematische Darstellung einer weiteren Ausführungsform

Figur 5 die schematische Anordnung einer Mehrfachinfusionseinrichtung.

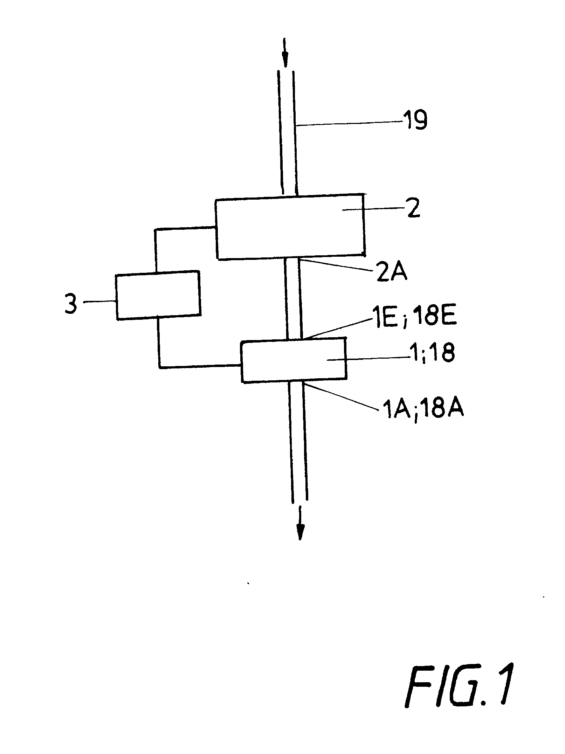

In der Figur 1 befindet sich in der vom Infusionsbehälter (nicht dargestellt) kommenden Flüssigkeitsleitung 19 ein tropfengeregelter Druckinfusionsapparat, der mit seinem Ausgang 2a an den Eingang 1E, 18E eines volumetrischen Druckinfusionsapparates 1 oder Durchflußmessers 18 angeschlossen ist. Die am Ausgang 1A bzw. 18A angeschlossene Flüssigkeitsleitung 20 führt zum Patienten. Die Apparate 2, 1, 18 sind elektrisch mit einer Auswerteeinheit 3 verbunden, die aus der an dem tropfengeregelten Druckinfusionsapparat ermittelten Tropfenzahl/Zeiteinheit und dem an dem volumetrischen Druckinfusionsapparat oder Durchflußmesser ermittelten Volumen/Zeiteinheit das Tropfenvolumen ermittelt.

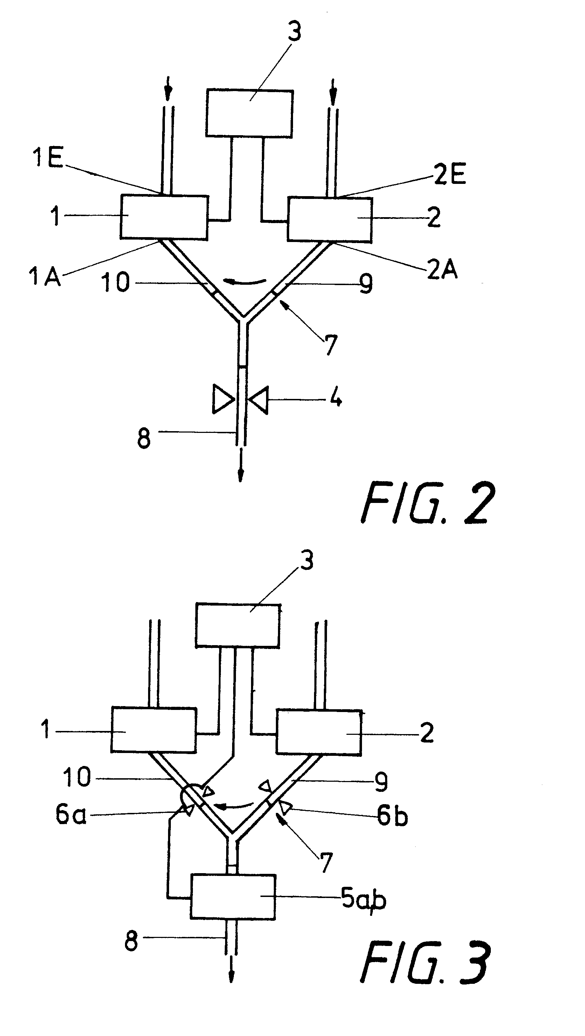

In der Figur 2 ist eine Anordnung mit einem volumetrischen Druckinfusionsapparat 1 und einem tropfengeregelten Druckinfusionsapparat 2 schematisch dargestellt. Der volumetrische Druckinfusionsapparat 1, der eine volumetrische Pumpe sein kann, ist mit seinem Ausgang 1A über ein Y-Stück 7 mit dem Ausgang 2A des tropfengeregelten Druckinfusionsapparates 2, der beispielsweise ein Tropfenregler sein kann, verbunden. Von diesem Abzweigstück 7 geht ein Schlauchstück als gemeinsame Flüssigkeitsleitung 8 ab. Die gezeigte Anordnung kann ein Teil eines Mehrfachinfusions-Systems ein, wobei dann die Apparate 1 und 2 mit ihren Eingängen 1E, 2E eingangsseitig an entsprechende nicht dargestellte Flaschen mit Infusionslösung angeschlossen sind. Die gemeinsame Leitung 8 stellt dann die Patientenleitung dar. Die beiden Apparate 1 und 2 sind an eine Auswerte-Einheit 3 elektrisch angeschlossen.

Zur Kalibrierung des Tropfenvolumens des tropfengeregelten Druckinfusionsapparates 2 wird die gemeinsame Flüssigkeitsleitung 8 mit Hilfe einer Klemme oder einem Ventil 4 geschlossen. Die volumetrisch arbeitende Pumpe 1 wird dann in umgekehrter Richtung betrieben, so daß die Flüssigkeit aus dem tropfengeregelten Zweig 9 in Pfeilrichtung in den Zweig 10 gesaugt wird. Die Auswerte-Einheit 3 erfasst die Tropfenzahl pro Zeiteinheit und das an der volumetrisch arbeitenden Pumpe eingestellte Volumen pro Zeiteinheit und ermittelt aus beiden Werten das Tropfenvolumen.

In der Figur 3 ist anstelle der Klemme 4 in der gemeinsamen Flüssigkeitsleitung 8 ein Fluß- oder Drucksensor 5 angeordnet.

Sowohl der volumetrische Druckinfusionsapparat 1 als auch der tropfengeregelte Druckinfusionsapparat 2 sind Pumpen, deren Förderraten zur Kalibrierung so eingestellt sind, daß die gesamte in den tropfengeregelten Zweig 9 geförderte Flüssigkeit in den Zweig 10 gelangt. Der Nettofluß in der gemeinsamen Flüssigkeitsleitung 8 wird damit Null, was vom Flußsensor 5a erkannt wird. Zur Steuerung der Förderraten kann die Auswerte-Einheit 3 zusätzlich noch mit dem Flußsensor 5a verbunden sein, so daß bei einer Strömungsänderung in der gemeinsamen Leitung 8 die beiden Pumpen 1 und 2 dann von der Auswerte-Einheit 3 entsprechend angesteuert werden können.

Wird in Figur 3 anstelle des Flußsensors 5a ein Druckmesser 5b eingesetzt, so läßt sich das Tropfenvolumen wie folgt kalibrieren. Zunächst wird die vorgegebene Tropfenzahl des tropfengeregelten Druckinfusionsapparates 2 eingestellt und der sich einstellende Druck am Drucksensor 5b gemessen. Der volumetrische Druckinfusionsapparat 1 ist ausgeschaltet bzw. durch eine Klemme 6a geschlossen. Anschließend wird die Pumpe 2 ausgeschaltet bzw. der Zweig 9 durch eine Klemme 6b geschlossen und der volumetrische Druckinfusionsapparat im Durchsatz so eingestellt, daß sich derselbe Druckabfall am Drucksensor 5b ergibt. Die Kalibrierung des Tropfenvolumens ist auf diese Weise dann möglich, wenn beide Flüssigkeiten in etwa die gleiche Viskosität besitzen, was bei wässrigen Lösungen der Fall ist.

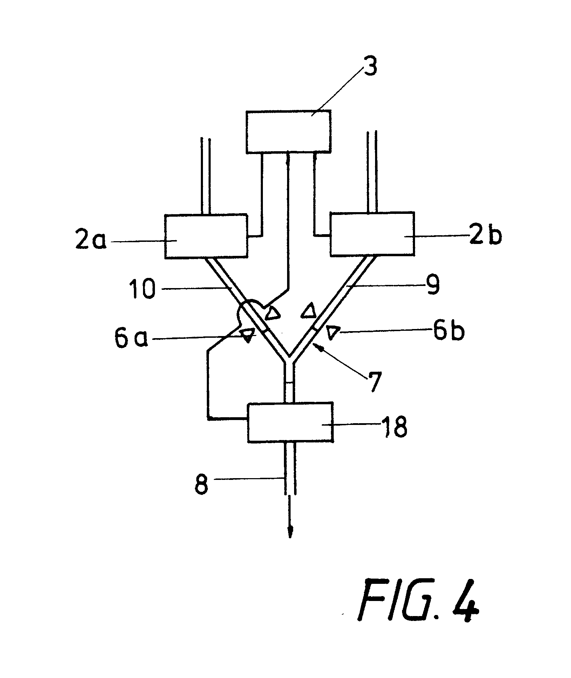

In der Figur 4 sind zwei tropfengeregelte Druckinfusionsapparate 2a, 2b über das Y-Stück 7 miteinander verbunden. In der gemeinsamen Leitung 8 ist ein Durchflußmesser 18 angeordnet. Zur Kalibrierung des tropfengeregelten Druckinfusionsapparates 2a wird der Zweig 9 mit Hilfe der Klemme oder des Ventils 6b unterbrochen. Anhand des durch den Durchflußmesser 18 strömenden Volumens pro Zeiteinheit, das von der Auswerte-Einheit 3 registriert wird, und der ebenfalls von der Auswerte-Einheit 3 gemessenen Tropfen~ate wird das Tropfenvolumen des tropfengeregelten Druckinfusionsapparates 2a ermittelt. Danach wird die Klemme bzw. das Ventil 6a im Zweig 10 geschlossen und die entsprechende Klemme bzw. Ventil 6b im Zweig 9 geöffnet, so daß auf die gleiche Weise auch das Tropfenvolumen des tropfengeregelten Druckinfusionsapparates 2b bestimmt werden kann.

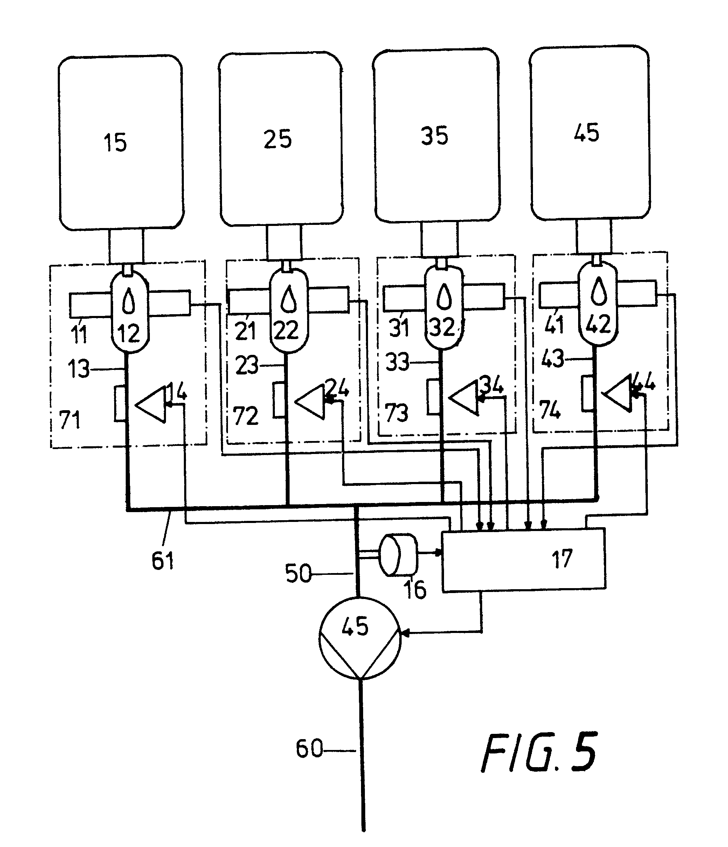

In der Figur 5 ist eine Mehrfachinfusionseinrichtung dargestellt, deren einzelne Kanäle tropfengeregelt sind, wobei lediglich eine einzige volumetrisch arbeitende Pumpe 45 zur Förderung herangezogen wird.

An die Flaschen 15, 25, 35, 45 mit Infusionslösungen sind jeweils Tropfenregler 71, 72, 73, 74 angeschlossen. Jeder Tropfenregler besteht aus Tropfensensoren 11, 21, 31 und 41, Tropfkammern 12, 22, 32 und 42, Leitungen 13, 23, 33, 43 sowie Klemmen 14, 24, 34, 44. Ausgangsseitig der Tropfenregler 71 bis 74 münden die Leitungen in ein Verteilerstück 61. Von diesem Verteilerstück 61, an dem auch ein Drucksensor 16 angeschlossen ist, führt eine Leitung 50 zur volumetrischen Infusionspumpe 45 und von dort zum Patienten.

Die Tropfensensoren 11 bis 41 sowie die Klemmen 14 bis 44 und der Tropfenregler 71 bis 74 sind mit einer Einheit 17 verbunden, die eine Steuereinheit und die Auswerte-Einheit 3 umfaßt. Außerdem ist an die Steuer-und Auswerte-Einheit 17 der Drucksensor 16 und die volumetrische Infusionspumpe 45 angeschlossen.

Vor der Kalibrierung wird das gesamte System entlüftet. Hierzu wird der Leitungsschlauch 50 nicht in die Pumpe 45 eingelegt. Die Klemmen der Regler 71 bis 74 werden nacheinander jeweils so lange geöffnet, bis der jeweilige Schlauch luftfrei mit Infusionslösung gefüllt ist. Bei einer pumpenunterstützten Füllung wird die Pumpe 45 durch das Steuergerät 17 so geregelt, daß sich stromauf der Pumpe 45 Atmosphärendruck einstellt. Die Klemme 14 des Reglers 71 wird geöffnet. Es wird dann solange Flüssigkeit gepumpt, bis ein stromab der Pumpe 45 üblicherweise vorhandener Luffdetektor (nicht dargestellt) Luftfreiheit anzeigt. Dann wird die Klemme 14 des Reglers 71 geschlossen und die Regler 72 bis 74 analog betrieben und somit entlüftet.

Die volumetrische Pumpe 45 wird anschließend zur Kalibrierung des Systems mit der für den Regler 71 vorprogrammierten volumetrischen Rate in Betrieb genommen. Die Klemme 14 des Reglers 71 wird nur langsam so weit geöffnet, bis sich am Drucksensor 16 stromauf der volumetrischen Pumpe 45 der für den Betrieb vorgesehene konstante Druck (vorzugsweise Atmosphärendruck) einstellt. Sobald Druckstabilität herrscht, wird die Tropfrate bestimmt und das Volumen pro Tropfen im Regler 71 in der Steuer- und Auswerte-Einheit 17 gespeichert. Die Kalibrierung erfolgt somit bei der im Betrieb vorgesehenen Rate, womit alle Einflüsse, die das Tropfenvolumen bestimmen können, berücksichtigt sind.

Danach erfolgt analog die Kalibrierung der Regler 72, 73 und 74. Natürlich kann auch der Ablauf Entlüften/Kalibrieren jeweils Regler für Regler erfolgen. Dann wird zunächst die Leitung des Reglers 71 entlüftet und dieser kalibriert, danach Regler 72 usw. Die Erfindung ist selbstverständlich nicht auf vier Regler beschränkt, sondern läßt sich beliebig erweitern.

Bezugszeichenliste

    1 volumetrischer Druckinfusionsapparat
    2 tropfengeregelter Druckinfusionsapparat
    2A Ausgang
    3 Auswerte-Einheit
    4 Klemme, Ventil
    5 Flußsensor, Drucksensor
    6a,b Klemme, Ventil
    7 Y-Stück
    8 gemeinsame Flüssigkeitsleitung
    9 tropfengeregelter Zweig
    10 Zweig
    11 Tropfensensor
    12 Tropfkammer
    13 Leitung
    14 Klemme
    15 Flasche
    16 Drucksensor
    17 Steuer- und Auswerte-Einheit
    18 Durchflußmesser
    19 Flüssigkeitsleitung
    20 Flüssigkeitsleitung
    21 Tropfsensor
    22 Tropfkammer
    23 Leitung
    24 Klemme
    25 Flasche
    31 Tropfensensor
    32 Tropfkammer
    33 Leitung
    34 Klemme
    35 Flasche

41 Tropfsensor
42 Tropfkammer
43 Leitung
44 Klemme
45 Pumpe
50 Leitung
60 Patientenleitung
61 Verteilerstück
71 Tropfenregler
72 Tropfenregler
73 Tropfenregler
74 Tropfenregler

**Ansprüche**

1. Verfahren zum Kalibrieren des Tropfenvolumens tropfengeregelter Druckinfusionsapparate, die in Kombination mit volumetrischen Druckinfusionsapparaten oder Durchflußmessern betrieben werden, dadurch gekennzeichnet,
daß die den tropfengeregelten Duckinfusionsapparat verlassende Flüssigkeit dem volumetrischen Druckinfusionsapparat oder dem Durchflußmesser zugeführt wird,
daß die während des Betriebs dieser Einrichtung an dem tropfengeregelten Druckinfusionsapparat ermittelte oder eingestellte Tropfenzahl/Zeiteinheit mit dem an dem volumetrischen Druckinfusionsapparat oder Durchflußmesser ermittelten oder eingestellten Volumen/Zeiteinheit verglichen wird, und
daß aus dem Vergleich auf das Tropfenvolumen geschlossen wird.

2. Verfahren nach Anspruch 1, bei dem der tropfengeregelte Druckinfusionsapparat und der volumetrisch arbeitende Druckinfusionsapparat jeweils ausgangsseitig an eine gemeinsame Flüssigkeitsleitung angeschlossen sind, dadurch gekennzeichnet, daß der volumetrische Druckinfusionsapparat in umgekehrter Richtung betrieben wird.

3. Verfahren nach Anspruch 2, dadurch gekennzeichnet, daß der Flüssigkeitsstrom in der gemeinsamen Flüssigkeitsleitung während der Kalibrierung unterbrochen wird.

4. Verfahren nach Anspruch 2, dadurch gekennzeichnet, daß die Durchflußraten des volumetrischen Druckinfusionsapparates und des tropfengeregelten Druckinfusionsapparates während der Kalibrierung so aufeinander abgestimmt werden, daß der Nettofluß in der gemeinsamen Flüssigkeitsleitung Null wird.

5. Verwendung des Verfahrens nach einem der Ansprüche 1 - 6 zur Kalibrierung der Tropfenvolumina tropfengeregelter Druckinfusionsapparate in Mehrfachinfusionseinrichtungen.

6. Anordnung zur Kalibrierung des Tropfenvolumens mindestens eines tropfengeregelten Druckinfusionsapparates, der in Kombination mit einem volumetrischen Druckinfusionsapparat oder einem Durchflußmesser betrieben wird, dadurch gekennzeichnet,
daß der Ausgang des tropfengeregelten Druckinfusionsapparates (2) an den Eingang oder an den Ausgang des volumetrischen Druckinfusionsapparates angeschlossen ist,
daß der tropfengeregelte Druckinfusionsapparat und der volumetrische Druckinfusionsapparat oder der Durchflußmesser elektrisch an eine Auswerteeinheit (3) angeschlossen sind, die zur Ermittlung des Tropfenvolumens aus der Tropfenzahl/Zeiteinheit und des Volumens/Zeiteinheit ausgebildet ist.

7. Anordnung nach Anspruch 6, dadurch gekennzeichnet, daß mindestens zwei tropfengeregelte Druckinfusionsapparate (2a, 2b) ausgangsseitig an eine gemeinsame Flüssigkeitsleitung (8) angeschlossen sind, und daß der volumetrische Druckinfusionsapparat oder der Durchflußmesser in der gemeinsamen Flüssigkeitsleitung (8) angeordnet ist.

8. Anordnung nach Anspruch 6, dadurch gekennzeichnet, daß der tropfengeregelte Druckinfusionsapparat (2) und der volumetrische Druckinfusionsapparat (1) ausgangsseitig an eine gemeinsame Flüssigkeitsleitung (8) angeschlossen sind und daß in der gemeinsamen Flüssigkeitsleitung (8) eine Absperreinrichtung (4) angeordnet ist.

9. Anordnung nach Anspruch 6, dadurch gekennzeichnet, daß der tropfengeregelte Druckinfusionsapparat (2) und der volumetrisch arbeitende Druckinfusionsapparat (1) ausgangsseitig an eine gemeinsame Flüssigkeitsleitung (8) angeschlossen sind und daß in der gemeinsamen Flüssigkeitsleitung (8) ein Fluß- oder ein Drucksensor (5) angeordnet ist.

FIG.1

*FIG. 2*

*FIG. 3*

FIG. 4

FIG.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 551 134 (SLAVIK et al.) <br> * Spalte 8, Zeilen 21-46; Figuren 1,2,5g * <br> --- | 1,6 | A 61 M 5/168 <br> G 01 F 25/00 |
| X | EP-A-0 266 716 (NIKKISO CO., LTD) <br> * Spalte 4, Zeilen 1-28; Figur 1 * <br> --- | 1,6 | |
| A | US-A-3 884 228 (HAHN) <br> * Spalte 6, Zeilen 5-15; Spalte 8, Zeilen 34-43; Figuren 1,3,5 * <br> --- | 1,6 | |
| A,D | DE-C-3 702 609 (B. BRAUN MELSUNGEN) <br> --- | | |
| A,D | DE-U-8 713 337 (B. BRAUN MELSUNGEN) <br> ----- | | |

**RECHERCHIERTE SACHGEBIETE (Int. Cl.5)**

A 61 M
G 01 F

Der vorliegende Recherchenbericht wurde für alle Patentansprüche erstellt

| Recherchenort | Abschlußdatum der Recherche | Prüfer |
|---|---|---|
| DEN HAAG | 18-10-1990 | CLARKSON P.M. |